# EUROPÄISCHE PATENTSCHRIFT

(11) **EP 3 477 650 B1**
(45) Veröffentlichungstag und Bekanntmachung des Hinweises auf die Patenterteilung: **23.11.2022**
(21) Anmeldenummer: 17198298.6
(22) Anmeldetag: 25.10.2017
(51) Int. Cl.: G16H 40/60, G16H 30/20, H04L 1/00, H04L 69/10, H04L 67/12

(54) **VERFAHREN UND EINRICHTUNG ZUR KOMMUNIKATION IN EINER MEDIZINISCHEN BILDGEBUNGSEINRICHTUNG UND MEDIZINISCHE BILDGEBUNGSEINRICHTUNG**
MEDICAL IMAGING DEVICE AND METHOD AND DEVICE FOR COMMUNICATION IN A MEDICAL IMAGING DEVICE
PROCÉDÉ ET DISPOSITIF DE COMMUNICATION DANS UN DISPOSITIF D'IMAGERIE MÉDICALE ET DISPOSITIF D'IMAGERIE MÉDICALE

(43) Veröffentlichungstag der Anmeldung: 01.05.2019
(73) Patentinhaber: Siemens Healthcare GmbH, 91052 Erlangen (DE)
(72) Erfinder: Baumgartl, Rudi, 91056 Erlangen (DE); Demharter, Nikolaus, 91077 Dormitz (DE)

(56) Entgegenhaltungen:
- CN-A- 106 301 659
- DE-A1-102006 052 437
- US-A- 3 599 160
- US-A1- 2009 137 898
- US-A1- 2016 174 928
- Anonymous: "Statistical time-division multiplexing - Wikipedia", , 11 July 2017 (2017-07-11), XP055389944, Retrieved from the Internet: URL:https://en.wikipedia.org/wiki/Statisti cal_time-division_multiplexing [retrieved on 2017-07-11]
- Itu-T: "ITU-T Interfaces for the optical transport network G.709/Y.1331", , 1 June 2016 (2016-06-01), XP055647169, Retrieved from the Internet: URL:https://www.itu.int/rec/dologin_pub.as p?lang=e&id=T-REC-G.709-201606-I!!PDF-E&ty pe=items [retrieved on 2019-11-27]
- P Nagasiva Kumar ET AL: "Analysis of Optical Time Division Multiplexing Using Packet Interleaving Scheme", International Journal of Scientific and Research Publications, 4 April 2013 (2013-04-04), XP055647205, Retrieved from the Internet: URL:http://www.ijsrp.org/research-paper-04 13/ijsrp-p1674.pdf [retrieved on 2019-11-27]
- GHARIYA RUTIKA ET AL: "Real time data transfer using fiber optic communication", 2016 10TH INTERNATIONAL CONFERENCE ON INTELLIGENT SYSTEMS AND CONTROL (ISCO), IEEE, 7 January 2016 (2016-01-07), pages 1-3, XP032989088, DOI: 10.1109/ISCO.2016.7726905 [retrieved on 2016-10-31]
- LIU JIANXIN ET AL: "The analysis and test of real-time performance for time-triggered CAN bus", AUTOMATION AND LOGISTICS, 2008. ICAL 2008. IEEE INTERNATIONAL CONFERENCE ON, IEEE, PISCATAWAY, NJ, USA, 1 September 2008 (2008-09-01), pages 3005-3009, XP031330185, ISBN: 978-1-4244-2502-0
- JIQIANG XIA ET AL: "Real-time and reliability analysis of time-triggered CAN-bus", CHINESE JOURNAL OF AERONAUTICS, vol. 26, no. 1, 16 January 2013 (2013-01-16), pages 171-178, XP055647309, AMSTERDAM, NL ISSN: 1000-9361, DOI: 10.1016/j.cja.2012.12.017

## Beschreibung

Die Erfindung betrifft ein Verfahren und eine Kommunikationseinrichtung zur Kommunikation zwischen wenigstens zwei Recheneinrichtungen einer medizinischen Bildgebungseinrichtung, insbesondere einer Magnetresonanzeinrichtung, über eine einzige Kommunikationsleitung. Daneben betrifft die Erfindung eine medizinische Bildgebungseinrichtung, insbesondere eine Magnetresonanzeinrichtung.

Heutige medizinische Bildgebungseinrichtungen bestehen aus einer Vielzahl von Komponenten, die der Steuerung, der Umsetzung von Peripheriefunktionen, der eigentlichen Bildgebung und weiteren Aufgaben dienen können. Viele dieser Komponenten weisen Recheneinrichtungen auf, die zum Betrieb der medizinischen Bildgebungseinrichtung mit anderen Recheneinrichtungen anderer Komponenten und/oder derselben Komponente kommunizieren, beispielsweise zur Ausgabe und Entgegennahme von Steuerbefehlen und/oder Bild- und/oder Messdaten. So wird in heutigen Magnetresonanzeinrichtungen bzw. Magnetresonanzanlagen meist ein zentraler Steuerrechner eingesetzt, der mit Recheneinrichtungen weiterer Komponenten kommuniziert, beispielsweise mit einer Peripherieeinheit, einer Sendeeinheit, einer Empfangseinheit und einer Gradienteneinheit. Die Peripherieeinheit kann wiederum mit Peripheriesubeinheiten kommunizieren; zudem kommunizieren die genannten Einheiten miteinander und/oder mit Erweiterungseinheiten, beispielsweise zusätzlichen Empfangseinheiten und dergleichen. Mithin sind die einzelnen Komponenten durch eine Vielzahl von Kabeln und Leitungen untereinander, aber auch mit dem zentralen Steuerrechner/ Bildberechnungscomputer verbunden. Die unterschiedlichen Komponenten bzw. deren Recheneinrichtungen weisen dabei unterschiedliche Anforderungen für die Informationsübertragung auf.

Beispielsweise sind als Kommunikationsinformationen Steuerinformationen mit und ohne Zeitbezug zu übertragen, wobei eine andere in der medizinischen Bildgebung häufige Anwendung isochrone Datenströme als Kommunikationsinformationen betrifft, bei denen aus der Taktfrequenz/ Taktrate wieder ein direkter Zeitbezug hergestellt werden soll. Die erstgenannten Kommunikationsinformationen werden typischerweise paketorientiert, die zweite Art von Kommunikationsinformationen typischerweise kanalorientiert, also über eine isochrone Direktkanalverbindung, übertragen.

Dies führt dazu, dass eine Vielzahl von Kommunikationsschnittstellen mit unterschiedlichen Kommunikationsprotokollen nebeneinander bestehen. Beispielsweise werden in modernen Magnetresonanzeinrichtungen als Kommunikationsprotokolle PCI express, ISO, RS 232, SPI, USB, CAN/CANopen und diverse spezialisierte proprietäre Protokolle eingesetzt.

Diese Vielzahl an Kommunikationsschnittstellen mit unterschiedlichen Kommunikationsprotokollen führt zu einem hohen technischen Aufwand, hohem Bauraum, komplexen Verkabelungen und somit insgesamt sehr hohen Kosten der medizinischen Bildgebungseinrichtung, insbesondere Magnetresonanzeinrichtung.

Die DE 102006052437 A1 und die DE 102008017819 B3 zeigen jeweils einen Aufbau eines MR-Steuerungssystems, die DE 102007058872 A1, die US 20090137898 A1, US 20160174928 A1 jeweils Vorrichtungen zur Datenübertragung in einem MR-System.

Aus der Druckschrift CN 106301659 A ist eine Übertragungseinrichtung für einen Magnetresonanztomographen bekannt. Die Übertragungseinrichtung weist eine Multiplexeinheit, eine serielle Übertragungseinheit und einen elektro-optischen Wandler auf. Die Übertragungseinheit ist ausgelegt, Magnetresonanzsignale mehrerer Kanäle zu multiplexen und gleichzeitig zu übertragen.

Die Druckschrift US 2009/137898 A1 beschreibt eine Vorrichtung zur Übertragung digitaler Signale in einer Magnetresonanzvorrichtung auf. In einer Lokalspule werden empfangene Magnetresonanzsignale verstärkt, von einem A/D-Wandler digitalisiert und mittels einer kapazitiven Kopplung übertragen.

Aus der Druckschrift US 2016/174928 A1 ist eine Vorrichtung und Verfahren zur Übertragung von Signalen in einem bildgebenden medizinischen System bekannt. Eine Übertragung von Signalen zwischen mindestens einer Empfangseinrichtung und einer Einrichtung des bildgebenden Systems erfolgt drahtlos über ein Funknetz.

Die Druckschrift DE 10 2006 052437 A1 beschreibt eine Magnetresonanzanlage mit einer analogen und einer digitalen Baugruppe. Die Digitalbaugruppe wird von einem Steuerrechner angesteuert und steuert wiederum die analoge Baugruppe im Rahmen einer Sequenz an. Zur Kommunikation und Synchronisation zwischen Steuerrechner und Digitalbaugruppe ist ein synchrones Netzwerk vorgesehen.

Aus der Druckschrift US 3599160 A ist ein digitales System und Verfahren zur Übertragung serieller Daten einer Vielzahl von Datenleitungen mit einem Multiplexer und einem Demultiplexer bekannt. Ein Prozessor steuert einen seriellen Eingangsdatenfluss von den Datenleitungen über einen Datenbus in einen zentralen Speicher. Eine fest verdrahtete Logik steht in Verbindung mit dem Prozessor, dem zentralen Speicher und dem Multiplexer, um den Beginn eines Datenworts zu erkennen und dann das Übertragen der seriellen Daten in den zentralen Speicher zu kontrollieren.

Die Druckschrift Anonymous: "Statistical time-division multiplexing - Wikipedia", 11. Juli 2017 (2017-07-11), XP055389944, Gefunden im Internet: URL:https://en.wikipedia.org/wiki/Statistical_timedivision_mu ltiplexing [gefunden am 2017-07-11] beschreibt ein statistisches Datenmultiplexing, bei dem einem einzelnen Kanal keine feste Bandbreite zugeordnet wird, sondern die Kanäle zur Reduktion der benötigten Bandbreite statistisch in einem paketorientierten Übertragungsmodus übertragen werden.

Aus der Druckschrift ltu-T: "ITU-T Interfaces for the optical transport network G.709/Y. 1331 ", 1. Juni 2016 (2016-06-01), XP055647169, [gefunden am 2019-11-27]sind Schnittstellen für optische Transportnetzwerke bekannt. Es werden eine Hierarchie des optischen Übertragungsnetzes, Funktion einer Datenstruktur für die Übertragung mittels mehrerer Wellenlängen, Datenrahmen, Bitraten und Formate zum Zuordnen von Anwendersignalen definiert.

Die Druckschrift P Nagasiva Kumar ET AL: "Analysis of Optical Time Division Multiplexing Using Packet lnterleaving Scheme", International Journal of Scientific and Research Publications, 4. April 2013 (2013-04-04), XP055647205, Gefunden im Internet: URL:http://www.ijsrp.org/researchpaper-0413/ijsrp-p1674.pdf [gefunden am 2019-11-27]beschreibt eine optische Datenübertragung im Zeitmultiplex. Es werden eine Mehrzahl an niederbitratigen Signalen in einem optischen Signal zusammengefasst. Die Druckschrift beschreibt eine Simulation einer verschachtelten Paketübertragung.

Aus der Druckschrift GHARIYA RUTIKA ET AL: "Real time data transfer using fiber optic communication", 2016 10TH INTERNATIONAL CONFERENCE ON INTELLIGENT SYSTEMS AND CONTROL (ISCO), IEEE, 7. Januar 2016 (2016-01-07), Seiten 1-3, XP032989088, DOI: 10.1109/ISC0.2016. 7726905 [gefunden am 201 6-1 0-31 ]ist eine gemeinsame Übertragung eines Audiostreams, Daten über Temperatur und Luftfeuchtigkeit und von Zeichenketten zwischen zwei Mikroprozessoren über eine Glasfaser bekannt.

Die Druckschrift LIU JIANXIN ET AL: "The analysis and test of real-time performance for time-triggered CAN bus", AUTOMATION AND LOGISTICS, 2008. ICAL 2008. IEEE INTERNATIONAL CONFERENCE ON, IEEE, PISCATAWAY, NJ, USA, 1. September 2008 (2008-09-01), Seiten 3005-3009, XP031330185, ISBN: 978-1-4244-2502-Obeschreibt eine Analyse und Test der Echtzeit-Leistungsfähigkeit einer CAN-Bus-Architektur im Zeitmultiplex-Verfahren TDMA.

Aus der Druckschrift JIQIANG XIA ET AL: "Real-time and Reliability analysis of timetriggered CAN-bus", CHINESE JOURNAL OF AERONAUTICS, Bd. 26, Nr. 1, 1. Februar 2013 (2013-02-01), Seiten 171-178, XP055647309, AMSTERDAM, NL ISSN: 1000-9361, DOI: 10.1016/j.cja.2012.12.017 ist ein Echtzeit-Betrieb eines CAN-Bus Systems im Zeitmultiplex TDMA bekannt. Ein Verfahren zur Zuteilung eines Zeitschlitzes wird verwendet, um die Übertragung eines zeitkritischen Signals mit einer sichergestellten maximalen Verzögerung bereitzustellen. Es wird die Verzögerung analysiert und eine maximale Verzögerung im schlechtesten Fall ermittelt.

Der Erfindung liegt daher die Aufgabe zugrunde, die Kommunikation innerhalb einer medizinischen Bildgebungseinrichtung hinsichtlich der unterschiedlichen Anforderungen für unterschiedliche Kommunikationsinformationen zu verbessern.

Diese Aufgabe wird erfindungsgemäß gelöst durch ein Verfahren zur Kommunikation zwischen wenigstens zwei Recheneinrichtungen einer Magnetresonanzeinrichtung, und eine Magnetresonanzeinrichtung gemäß den unabhängigen Ansprüchen. Vorteilhafte Ausgestaltungen ergeben sich aus den Unteransprüchen.

In einem erfindungsgemäßen Verfahren zur Kommunikation zwischen wenigstens zwei Recheneinrichtungen einer medizinischen Bildgebungseinrichtung, einer Magnetresonanzeinrichtung, über eine einzige Kommunikationsleitung ist vorgesehen, dass über jeweils einen eigenen Dateneingang erhaltene, unterschiedlichen Kommunikationsarten zugeordnete Kommunikationsinformationen in über die Kommunikationsleitung zu übertragende Datenstrukturen definierter Größe zusammengeführt werden, wobei die Datenstrukturen zyklisch um eine Zykluszeit beabstandet über die Kommunikationsleitung übertragen werden, und Kommunikationsinformationen der unterschiedlichen Kommunikationsarten aus über die Kommunikationsleitung empfangenen Datenstrukturen extrahiert und an den jeweiligen Kommunikationsarten zugeordnete Datenausgänge ausgegeben werden, wobei die Kommunikationsarten eine erste, paketorientierte Kommunikationsart und eine zweite, isochrone, einer isochronen Direktkanalkommunikation entsprechende Kommunikationsart umfassen.

Die konkrete Ausgestaltung der als Magnetresonanzeinrichtung ausgebildeten erfindungsgemäßen medizinischen Bildgebungseinrichtung sieht vor, dass diese eine zentrale Steuereinrichtung als erste Recheneinrichtung, die insbesondere in einem Technikraum und/oder einem Bedienraum außerhalb eines geschirmten Magnetresonanzraums angeordnet ist, Recheneinrichtungen wenigstens einer Gradienteneinheit, wenigstens einer Sendeeinheit, wenigstens einer Empfangseinheit und wenigstens einer Peripherieeinheit als zweite Recheneinrichtungen und Recheneinrichtungen von wenigstens einer Peripheriesubeinheit als dritte Recheneinrichtungen aufweist, wobei die erste Recheneinrichtung, insbesondere als Master, jeweils über eine der wenigstens einen Kommunikationseinrichtung mit den zweiten Recheneinrichtungen, insbesondere als Slave, verbunden ist und wobei die Peripherieeinheit, insbesondere als Master, über jeweils eine der wenigstens einen Kommunikationseinrichtung mit den dritten Recheneinrichtungen, insbesondere als Slave, verbunden ist.

Auf diese Weise wird auf einer bekannten Architektur von Magnetresonanzeinrichtungen aufgesetzt, wobei die bisher genutzten Kommunikationsschnittstellen, beispielsweise PCI Express, USB, RS 232, SPI, proprietäre Protokolle, ..... jeweils durch die hier beschriebene Kommunikationsschnittstelle bzw. das entsprechende Kommunikationsprotokoll (UCI) ersetzt werden. Insbesondere kann in konkreter Weiterbildung vorgesehen sein, dass die zweiten Recheneinrichtungen wenigstens teilweise untereinander durch wenigstens eine der wenigstens einen Kommunikationseinrichtung verbunden sind. Auch ein "horizontaler" Datenverkehr, insbesondere in einem Ring, kann mithin mittels der hier vorgeschlagenen Kommunikationsschnittstelle bereitgestellt werden.

Gemäß dem erfindungsgemäßen Verfahren ist es vorgesehen, dass wenigstens ein vorbestimmter Unteranteil der Datenstruktur, insbesondere eines der Wahlfreier-Zugriff-Kommunikation zugeordneten Anteils, zum Übermitteln eines Interrupts, insbesondere von einem Slave an einen Master, verwendet wird.

Während seitens eines Masters beliebige Informationen an einen Slave weitergegeben werden können, ist dies in der Gegenrichtung schwieriger umzusetzen. Liegt seitens des Slave ein Kommunikationsbedürfnis mit dem Master vor, beispielsweise eine eine weitere Kommunikation temporär unmöglich machende Bedingung, ist vorgesehen, dass der Slave den vorbestimmten Unteranteil der Datenstruktur nutzt, um einen Interrupt zu übermitteln, wobei als Reaktion der Master die Kommunikation beispielsweise pausieren kann und eine entsprechende Abfragenachricht bezüglich des eingetretenen Umstands an den Slave schicken kann. Bevorzugt ist hierbei ein Unteranteil, beispielsweise Slot, der Datenstruktur (frame), der der Wahlfreier-Zugriff-Kommunikation zugeordnet ist, vorbelegt, da hier ohnehin eine langsamere, geringere Datenmengen betreffende Kommunikationsart gegeben ist.

Die erfindungsgemäße medizinische Bildgebungsvorrichtung mit mehreren Recheneinrichtungen weist eine Kommunikationseinrichtung zur Kommunikation zwischen wenigstens zwei Recheneinrichtungen einer medizinischen Bildgebungseinrichtung, insbesondere einer Magnetresonanzeinrichtung, weist auf:
- eine Kommunikationsleitung, und
- für jede Recheneinrichtung jeweils eine Kommunikationsschnittstellenbaugruppe, die mit der Kommunikationsleitung verbunden ist,
wobei die Kommunikationsschnittstellenbaugruppen jeweils eine Trennungs- und Zusammenführungseinheit zum Zusammenführen von über jeweils einen eigenen Dateneingang erhaltenen, unterschiedlichen Kommunikationsarten zugeordneten Kommunikationsinformationen in über die Kommunikationsleitung zu übertragende Datenstrukturen einer definierten Größe sowie zum zyklischen, um eine Zykluszeit beabstandeten Übertragen der Datenstrukturen über die Kommunikationsleitung, und zum Extrahieren von Kommunikationsinformationen der unterschiedlichen Kommunikationsarten aus über die Kommunikationsleitung empfangenen Datenstrukturen der jeweils anderen Kommunikationsschnittstellenbaugruppe sowie zur Weitergabe an den jeweiligen Kommunikationsarten zugeordnete Datenausgänge, wobei an ein einer ersten, paketorientierten Kommunikationsart zugeordnetes Dateneingang-Datenausgang-Paar eine Paketkommunikationseinheit zur Bereitstellung und Entgegennahme von Kommunikationsinformationen der ersten Kommunikationsart und an ein einer zweiten, einer isochronen Direktkanalkommunikation entsprechenden Kommunikationsart zugeordnetes Dateneingang-Datenausgang-Paar eine isochrone Daten als Kommunikationsinformation bereitstellende und entgegennehmende isochrone Kommunikationseinheit angeschlossen ist. Mithin ist die Kommunikationseinrichtung zur Ausführung des erfindungsgemäßen Verfahrens ausgebildet.

Die erfindungsgemäße medizinische Bildgebungsvorrichtung weist eine zentrale Steuereinrichtung als erste Recheneinrichtung, die insbesondere in einem Technikraum und/oder einem Bedienraum außerhalb eines geschirmten Magnetresonanzraums angeordnet ist, Recheneinrichtungen wenigstens einer Gradienteneinheit, wenigstens einer Sendeeinheit, wenigstens einer Empfangseinheit und wenigstens einer Peripherieeinheit als zweite Recheneinrichtungen und Recheneinrichtungen von wenigstens einer Peripheriesubeinheit als dritte Recheneinrichtungen aufweist, wobei die erste Recheneinrichtung, insbesondere als Master, jeweils über eine der wenigstens einen Kommunikationseinrichtung mit den zweiten Recheneinrichtungen, insbesondere als Slave, verbunden ist und wobei die Peripherieeinheit, insbesondere als Master, über jeweils eine der wenigstens einen Kommunikationseinrichtung mit den dritten Recheneinrichtungen, insbesondere als Slave, verbunden ist.

Dabei sei angemerkt, dass im Rahmen der vorliegenden Erfindung eine Recheneinrichtung selbstverständlich auch als eine Steuerungseinrichtung dienen kann.

Erfindungsgemäß wird zusammenfassend eine medizinische Bildgebungsvorrichtung mit einer neuartigen Kommunikationsschnittstelle bereitgestellt, die ein Kommunikationsprotokoll realisiert, das als UCI (unified communication interface) bezeichnet werden kann. UCI ist ein serielles Punkt-zu-Punkt-Kommunikationsprotokoll, welches wenigstens zwei unterschiedliche Kommunikationsarten durch Zeitaufteilungs-Multiplexing realisiert, bevorzugt unter zusätzlicher Verwendung von Sicherungsmechanismen. Das bedeutet, jeder Kommunikationsart ist ein festgelegter Anteil (wenigstens ein Slot) der Datenstruktur (frame) festgelegter, unveränderbare Größe/Länge zugeordnet. Die Kommunikationsinformationen der unterschiedlichen Kommunikationsarten, die entsprechend von höhergelegenen Schichten, insbesondere der Paketkommunikationseinheit und der Isochronkommunikationseinheit, die der Transportschicht im ISO-Modell zuzuordnen sind, bereitgestellt werden, werden mithin genutzt, um die festgelegten Anteile der Datenstruktur fester, vorgegebener Länge bzw. Größe aufzufüllen, was innerhalb der Trennungs- und Zusammenführungseinheit geschieht. Dabei kann die Trennungs- und Zusammenführungseinheit beispielsweise als "transmit and receive data interface" bezeichnet werden, bei der bevorzugten Verwendung einer Master-Slave-Architektur mithin beispielsweise als "transmit and receive data interface slave" (TARDIS) bzw. "transmit and receive data interface master" (TARDIM).

Es ist vorgesehen, dass eine Trennungs- und Zusammenführungseinheit als ein Master ausgebildet ist und die wenigstens eine weitere der Trennungs- und Zusammenführungseinheiten als ein Slave. Mithin wird eine Master-Slave-Architektur verwendet, bei der einer der Kommunikationspartner als ein Master, der andere als ein Slave agiert, worauf im Folgenden noch genauer eingegangen werden wird. Entsprechende Trennungs- und Zusammenführungseinheiten können dabei bereits in ihrer Hardware auf die Funktion als Master oder Slave ausgestaltet werden. Der Master kann hierbei die folgenden Aufgaben haben: Initialisierung und Antwort des Aufbaues der Kommunikationsverbindung, Initialisierung der paketorientierten Kommunikation, Senden und Empfangen der isochronen Kommunikationsinformationen. Für den Slave können die Aufgaben der Initialisierung und Antwort zum Aufbau der Kommunikationsverbindung, der Antwort zur paketorientierten Kommunikation sowie des Sendens und Empfangens der isochronen Kommunikationsdaten vorgesehen sein. Dabei sei an dieser Stelle angemerkt, dass es durchaus denkbar ist, zwischen zwei als Slave ausgebildeten Trennungs- und Zusammenführungseinheiten eine isochrone Kommunikation herzustellen. In letzterem Fall initialisieren die Slaves den Aufbau der Kommunikationsverbindung gemeinsam.

Insgesamt ermöglicht die hier beschriebene Kommunikationsschnittstelle also eine Zusammenfassung und Vereinheitlichung der Kommunikationspfade innerhalb einer medizinischen Bildgebungseinrichtung, die sowohl eine schnelle, paketorientierte Kommunikation als auch eine isochrone Kommunikation bereitstellt, um die benötigten Kommunikationsinformationen zwischen den beteiligten Komponenten bzw. deren Recheneinrichtungen auszutauschen. Paketorientierte und kanalorientierte, isochrone Übertragung wird auf einer Übertragungsstrecke, nämlich der Kommunikationsleitung, kombiniert, um die Kommunikationsschnittstellen auf ein Minimum (nämlich eine) zu reduzieren. Auf diese Weise werden der Aufwand, die Komplexität und die Kosten der Kommunikation in der medizinischen Bildgebungseinrichtung reduziert. Insbesondere sinken nicht nur die Herstellungskosten, sondern auch die Wartungskosten für die medizinischen Bildgebungseinrichtungen, da die Verkabelung stark vereinfacht wird. Dadurch, dass zwischen verschiedenen Paaren von Recheneinrichtungen dieselbe Kommunikationsschnittstelle bzw. dasselbe Kommunikationsprotokoll verwendet werden kann, sinkt auch der Entwicklungsaufwand, da dasselbe Schnittstellendesign in allen Komponenten bzw. für alle Paare von Recheneinrichtungen zum Einsatz kommen kann.

Dabei sei an dieser Stelle noch angemerkt, dass die Trennung/ Zusammenführung der Kommunikationsinformationen gemäß dem OSI-Schichtmodell im Data Link Layer erfolgt, dem mithin die Trennungs- und Zusammenführungseinheit zuzuordnen ist. Mithin ist das hier eingeführte Kommunikationsprotokoll transparent für verwendete Software, so dass die bereits vorgesehenen und entwickelten Softwarekomponenten der Recheneinrichtungen nicht angepasst werden müssen.

In einer besonders bevorzugten Ausgestaltung der vorliegenden Erfindung ist vorgesehen, dass als erste Kommunikationsart eine Streaming-Kommunikation und/oder eine Wahlfreier-Zugriff-Kommunikation zum Abfragen wenigstens eines Speicherregisters wenigstens einer der Recheneinrichtungen verwendet werden. Bevorzugt werden mithin drei Protokolle bzw. Kommunikationsarten vereint, nämlich eine Streaming-Kommunikation, eine Wahlfreier-Zugriff-Kommunikation (random access communication) und eine isochrone Kommunikation. In dieser Ausgestaltung stellt mithin die Kommunikationsschnittstelle bereit:
- ein Random-Access-Interface für niedrige Zugriffslatenz,
- ein Streaming-Interface für hohen Datendurchsatz und
- ein isochrones Interface für konstante Datenübertragungslatenz.

Der wahlfreie Zugriff erfolgt dabei über adressierbare Register und Speicherbereiche. In der Master-Slave-Architektur wird die Kommunikation dabei von einem Master initiiert und von einem adressierten Slave beantwortet. Dabei kann in einer ersten, einfachen Ausgestaltung vorgesehen sein, dass ein Zugriff erst komplettiert werden muss, bevor ein zweiter Zugriff beginnen kann. Vorgesehen sein kann jedoch auch, dass eine parallele Handhabung mehrerer Anfragen in der Wahlfreier-Zugriff-Kommunikation erfolgt. Das bedeutet, es ist auch eine Unterstützung von sogenannten "Split Transactions" denkbar. Der durch die Wahlfreier-Zugriff-Kommunikation (random access communication) bereitgestellte Service wird in der Regel für den Zugriff auf einzelne Register genutzt.

Mittels der Streaming-Kommunikation wird direkter Datenfluss zwischen dedizierten Datenquellen und Datensenken als weiterer Service bereitgestellt (streaming access communication). Dieser Datenkanal wird bevorzugt im Hinblick auf die paketorientierte Kommunikation genutzt, welche von höheren Protokoll-Schichten, insbesondere der Transportschicht im OSI-Modell, bereitgestellt wird. Dieser Service unterliegt bevorzugt einer Flusskontrolle zur Vermeidung von FIFO-Über- und - Unterläufen. Die Streaming-Kommunikation wird in der Regel zur Übertragung von Massendaten als Kommunikationsinformation genutzt. Die Streaming-Kommunikation ist bevorzugt symmetrisch und voll-duplex-bidirektional. Das bedeutet, dass eine Recheneinrichtung an einer Kommunikationsschnittstellenbaugruppe gleichzeitig Datenquelle und Datensenke sein kann.

Schließlich wird eine isochrone Datenübertragung angeboten, wobei dieser Kommunikationsservice im Wesentlichen einem Bündel von Drähten, die Signale parallel und zeitlich gleichförmig (isochron) übertragen, entspricht. So wird eine konstante Datenrate gewährleistet, nachdem die Daten in Echtzeit fließen. Die Datenquelle und die Datensenke müssen jederzeit in der Lage sein, die Kommunikationsinformation rechtzeitig zu liefern und rechtzeitig entgegenzunehmen. Ein typischer Anwendungsfall sind Magnetresonanz-Echtzeitdaten, insbesondere die Übertragung von ADC-Daten oder DAC-Daten als Kommunikationsinformation, die im Einzelfall auch vor- oder nachverarbeitet werden können.

Wie bereits erwähnt, wird die paketorientierte Kommunikation zwischen den Recheneinrichtungen durch eine insbesondere einer Transportschicht zugeordnete, mit dem Dateneingang und dem Datenausgang der entsprechenden Kommunikationsart verbundene Paketkommunikationseinheit bereitgestellt. Dabei kann konkret vorgesehen sein, dass die paketorientierte Kommunikation wenigstens teilweise nach dem TCP/IP-Protokoll und/oder einem proprietären Protokoll und/oder dem CAN-Protokoll und/oder dem CANopen-Protokoll erfolgt. Selbstverständlich sind auch andere Protokolle grundsätzlich denkbar, deren Kommunikationsinformation über die wenigstens eine Paketkommunikationseinheit bereitgestellt wird.

Insbesondere bei einer Magnetresonanzeinrichtung als medizinische Bildgebungseinrichtung ist es innerhalb des Magnetresonanzraums (Schirmkabine) vorteilhaft, wenn eine optische Kommunikationsleitung als die Kommunikationsleitung verwendet wird, wobei die Datenstrukturen beidseitig durch Optokoppler umgewandelt werden. Die optische Signalübertragung hat nicht nur den Vorteil der hohen Übertragungsgeschwindigkeit, sondern ist zudem störungsfrei hinsichtlich sonstiger elektromagnetischer Felder, insbesondere der Magnetfelder, die im Rahmen der Magnetresonanzbildgebung angewendet werden. Der Trennungs- und Zusammenführungseinheit ist dann ein Optokoppler, also ein optisch-elektrischer Wandler, vorzuschalten, um die zu verarbeitenden elektronischen Signale zu erhalten bzw. entsprechend in optische, zu übertragende Signale umzusetzen.

Eine Weiterbildung der Erfindung sieht vor, dass für jede Kommunikationsart ein eigener Taktgeber verwendet wird, dessen Takt insbesondere von einer Master-Clock (Master-Taktgeber), einer jeweiligen Kommunikationsschnittstellenbaugruppe abgeleitet wird. Das bedeutet, die verschiedenen Kommunikationsarten können mit eigenen, insbesondere applikationsspezifisch zu wählenden Takten betrieben werden, wozu beispielsweise innerhalb der Trennungs- und Zusammenführungseinheit oder aber innerhalb entsprechender Paketkommunikationseinheiten/Isochronkommunikationseinheiten ein entsprechender Taktgeber vorliegen kann. Dieser Taktgeber leitet bevorzugt seinen Takt von einem Grundtakt einer Master-Clock (Master-Taktgeber) ab, was insbesondere im Rahmen der Magnetresonanz wiederum zweckmäßig ist, da dann eine Grundfrequenz als Grundtakt der Master-Clock gewählt werden kann, der und dessen Vielfache möglichst wenig mit der Magnetresonanzbildgebung interferieren. Als eine vorteilhafte Wahl hat sich hierbei ein Grundtakt bzw. Systemtakt von 2,5 MHz für die Master-Clock erwiesen, worauf bezüglich der Ausbildung der medizinischen Bildgebungseinrichtung als Magnetresonanzeinrichtung nochmals näher eingegangen werden wird.

Wie bereits erwähnt, ist die Trennungs- und Zusammenführungseinheit zum zyklischen Aussenden einer aktuellen Datenstruktur nach jeweils einer Zykluszeit ausgebildet. Je nach der gewünschten Datenübertragungsrate können dabei unterschiedliche Zykluszeiten auftreten, beispielsweise eine Zykluszeit von 200 ns bei 5 GBit/s und eine Zykluszeit von 400 ns bei 2,5 GBit/s. Im Rahmen der vorliegenden Erfindung ist mithin sichergestellt, dass immer um eine Zykluszeit beabstandet eine Datenstruktur ausgesendet wird und immer um eine Zykluszeit beabstandet eine Datenstruktur empfangen wird. Nachdem die Datenstruktur in verschiedene Anteile aufgeteilt ist, wobei zumindest ein Teil der Anteile mit Kommunikationsinformationen der unterschiedlichen Kommunikationsarten befüllt werden, und zudem diese Anteile festgelegt sind, äußert sich dies auch in entsprechenden Zeitabständen/Zeitfenstern, in denen die Anteile beim Sender abgeschickt bzw. beim Empfänger empfangen werden. Mithin können unterschiedlichen Anteilen der Datenstruktur unterschiedliche Zeitfenster der Zykluszeit hinsichtlich der Übertragung zugeordnet sein. Dies ist insbesondere im Hinblick auf die isochrone Kommunikation nützlich.

Zweckmäßigerweise wird die Datenstruktur zu deren Beginn mit einem Header versehen, wie dies grundsätzlich im Stand der Technik bekannt ist. Der Anteil des Headers an der Datenstruktur kann hierbei eher klein gewählt werden, beispielsweise in einer Größe von 4 Byte. Dem Header, der auch als Präambel bezeichnet werden kann, folgen hierbei die Nutzdaten, also die Payload, die zumindest im Data Link Layer wiederum unterteilt definiert ist, wobei zumindest ein Teil dieser Anteile (Slots) den jeweiligen Kommunikationsarten fest zugeordnet ist. Der Header kann auch, in eingeschränkter Form, zusätzlich zur Übermittlung von Informationen eingesetzt werden.

In einer besonders bevorzugten Weiterbildung der vorliegenden Erfindung ist vorgesehen, dass eine Vorwärtsfehlerkorrektur (forward error correction - FEC) erfolgt, wobei eine insbesondere auf alle Kommunikationsinformationen aller Kommunikationsarten bezogene Vorwärtsfehlerkorrekturinformation erzeugt und in einen Vorwärtsfehlerkorrekturanteil der Datenstruktur eingefügt wird, wobei die Vorwärtsfehlerkorrekturinformation bei Empfang jeder Datenstruktur ausgewertet wird. Auf diese Weise wird eine hohe Übertragungssicherheit der hier beschriebenen Kommunikationsschnittstelle bzw. des hier beschriebenen Kommunikationsprotokolls sichergestellt. Die Vorwärtsfehlerkorrektur ist eine Technik, die dazu dient, die Fehlerrate bei der Übertragung der Kommunikationsinformationen zu senken, wobei der Sender die zu übertragenden Daten durch Hinzufügen der Vorwärtsfehlerkorrekturinformation redundant kodiert, so dass der Empfänger Übertragungsfehler ohne Rückfrage beim Sender erkennen und korrigieren kann. Die redundante Kodierung bzw. die Auswertung, welche zu einer Korrektur und/oder, bei nicht möglicher Korrektur, zu einem Kommunikationsabbruch führen kann, werden bevorzugt durch eine Vorwärtsfehlerkorrektureinrichtung der entsprechenden Trennungs- und Zusammenführungseinheiten durchgeführt.

Insgesamt kann die Datenstruktur im Data Link Layer mithin als den Kommunikationsinformationen der unterschiedlichen Kommunikationsarten zugeordnete Anteile aufgefasst werden, die von einem Header und einem Vorwärtsfehlerkorrekturanteil eingerahmt sind. Dabei sei darauf hingewiesen, dass die Vorwärtsfehlerkorrektur vorliegend ohnehin eine zweckmäßige Wahl darstellt, da eine Korrektur von Übertragungsfehlern "on the fly" möglich ist, ohne dass ein wiederholtes Senden notwendig wäre. Denn ein derartiges wiederholtes Senden wäre aufgrund der Isochronität zumindest des entsprechenden Anteils der Kommunikationsinformation auch nicht zulässig. Durch die Vorwärtsfehlerkorrektur wird auch ein erheblicher Vorteil gegenüber anderen Protokollen geliefert, bei denen keine Korrektur gestörter Daten oder eine wiederholte Übertragung gestörter Daten vorgesehen ist.

Vorzugsweise kann der Aufbau einer Kommunikationsverbindung zwischen den Recheneinrichtungen durch einen Aushandlungsvorgang zu Beginn einer Kommunikationssitzung erfolgen. Derartige Aushandlungsvorgänge sind grundsätzlich bereits bekannt. Insbesondere kann der erwähnte Header der Datenstruktur diese erfolgreiche Aushandlung bestätigen. Eine automatisierte Ausführung eines derartigen Aushandlungsvorgang kann auch "hot plugging" unterstützen. Das bedeutet, nachdem die Recheneinrichtungen und somit die Kommunikationsschnittstellenbaugruppen eingeschaltet wurden, aber auch nachdem die Kommunikationsleitung, beispielsweise als Lichtwellenleiter, gesteckt wurde, findet eine Selbstinitialisierung in einen sicheren Modus statt. Im Beispiel der bevorzugten Master-Slave-Ausgestaltung kann der Master die Kommunikationsverbindung in verschiedenen Schritten aufbauen, wobei beispielsweise das Aushandeln des Aufbaus der Kommunikationsverbindung einen Trainingsvorgang unter Austausch von Basiskommunikationsdaten umfassen kann. Denkbar ist es jedoch auch, den Aushandlungsvorgang symmetrisch zwischen Master und Slave zu betreiben, indem jeder der beiden Teilnehmer seinem Kommunikationsschnittstellenpartner den eigenen Zustand mitteilt und die Gegenstelle zum nächsten Schritt auffordert.

In einer konkreten Ausgestaltung können beispielsweise beide Trennungs- und Zusammenführungseinheiten, insbesondere also der Master und der Slave, regelmäßig ein Grundsignal, insbesondere ein Byte-Alignment-Signal, aussenden. Bei jeweiligem Empfang ist bekannt, dass beide Trennungs- und Zusammenführungseinheiten in Betrieb sind und gerade keine Kommunikationsverbindung betreiben. Einem Byte-Alignment kann dann ein Word-Alignment folgen, wobei beim sogenannten Wordlock die Kommunikationsverbindung als hergestellt betrachtet werden kann und Basisstrukturen, also Frames, übertragen werden können. Selbstverständlich sind auch andere Aushandlungsvorgänge im Rahmen der vorliegenden Erfindung einsetzbar.

Es sei angemerkt, dass ein solcher Aushandlungsvorgang, der auch als Initialisierungssequenz bezeichnet werden kann, auch zum Unterbrechen und zum Neuaufbau einer Kommunikationsverbindung insbesondere ohne Ausschalten der Kommunikationspartner, beispielsweise für Servicezwecke, dienen kann.

Eine nicht beanspruchte Ausführungsform der hier beschriebene Kommunikationsschnittstelle kann nicht nur zur Verbindung zweier Komponenten der medizinischen Bildgebungseinrichtung eingesetzt werden, sondern auch innerhalb einer Komponente, zwischen Subkomponenten oder sogar innerhalb von Baueinheiten, beispielsweise auf einer Leiterplatte zur Kommunikation zwischen zwei Chips, beispielsweise FPGAs und/oder Mikrochips.

Im Rahmen der vorliegenden Erfindung ist ein sogenanntes "Daisy Chaining" vorgesehen. Dabei wird eine Reihe von Komponenten bzw. Recheneinrichtungen in Serie verbunden, wobei dennoch eine Kommunikation zwischen all diesen Recheneinrichtungen möglich sein soll. In diesem Fall sieht eine zweckmäßige Weiterbildung der Erfindung vor, dass bei Kommunikation zwischen zwei Recheneinrichtungen über eine zwischengelagerte Recheneinrichtung durch diese empfangene Datenstrukturen und/oder Kommunikationsinformationen zu Kommunikationszielen zugeordnet werden und an die Kommunikationsziele bei nicht der eigenen Recheneinrichtung entsprechendem Kommunikationsziel weitergeleitet werden. Konkret kann vorgesehen sein, dass wenigstens die Kommunikationsschnittstellenbaugruppe der zwischengelagerten Recheneinrichtung eine Arbitrierungseinrichtung zur Zuordnung von Datenstrukturen und/oder Kommunikationsinformationen zu Kommunikationszielen und zur Weiterleitung an die Kommunikationsziele bei nicht der eigenen Recheneinrichtung entsprechendem Kommunikationsziel aufweist. Dabei ist es selbstverständlich auch möglich, dass eine Kommunikationsinformation und/oder Datenstruktur mehrere Kommunikationsziele hat, mithin sowohl in der zwischengelagerten Recheneinrichtung als auch in einer in der Serie anschließenden Recheneinrichtung verwertet werden können. So ist es beispielsweise denkbar, isochrone Datenströme an zwei oder mehr Recheneinrichtungen zu leiten. Die Kommunikationsziele können insbesondere Teil der Kommunikationsinformation sein, so dass beispielsweise beim Trennen von Kommunikationsinformationen in einer Datenstruktur die Kommunikationsziele identifiziert werden und bei nicht der eigenen Recheneinrichtung entsprechendem Kommunikationsziel die Kommunikationsinformationen in einer weiterzuleitenden Datenstruktur durch die oder eine weitere Trennungs- und Zusammenführungseinheit eingefügt werden.

Bevorzugt ist die Kommunikationseinrichtung zur Kommunikation mit einer Kommunikationsrate von wenigstens 1 Gbit/s ausgebildet. Beispielsweise können Varianten von Kommunikationsschnittstellenbaugruppen erzeugt werden, die für eine Kommunikationsgeschwindigkeit von 2,5 Gbit/s oder eine Kommunikationsgeschwindigkeit von 5 Gbit/s ausgelegt sind. Entsprechende Zeitzyklen können sich beispielsweise als 400 ns bzw. 200 ns ergeben. Denkbar ist es im Rahmen der vorliegenden Erfindung auch, dass die Kommunikationsschnittstellenbaugruppen mit unterschiedlichen, vorgegebenen Kommunikationsraten betreibbar und zur Aushandlung einer Kommunikationsrate zu Beginn einer Kommunikationssitzung ausgebildet sind. Auch eine Bereitstellung unterschiedlicher Übertragungsraten über dieselbe Kommunikationsleitung, welche dann bei Bedarf konfiguriert wird, kann also möglich sein, was insbesondere dann zweckmäßig ist, wenn die Kommunikationsschnittstellenbaugruppen universell für verschiedene Kommunikationsstrecken hergestellt und eingesetzt werden sollen, mithin nicht gezielt für bestimmte Aufgaben/Applikationen/Recheneinrichtungen bereitgestellt werden.

Die Kommunikationsschnittstellenbaugruppen, konkret die Trennungs- und Zusammenführungseinheiten, können als integrierte Schaltung (ASIC) und/oder FPGA und/oder als Teil eines FPGA realisiert sein. Dabei sieht eine besonders vorteilhafte Weiterbildung der vorliegenden Erfindung vor, dass die Kommunikationsschnittstellenbaugruppen durch einen recheneinrichtungsspezifischen FPGA realisiert sind, der wenigstens einen Teil der Rechenleistung der Recheneinrichtung bereitstellt. Durch eine derartige Kombination der Kommunikationsschnittstellenfunktionalität mit weiteren Funktionalitäten der Recheneinrichtung können weitere Bausteine, beispielsweise weitere FPGAs, eingespart werden, was die Herstellungskosten deutlich reduzieren kann. Die entsprechenden FPGAs werden dann recheneinrichtungsspezifisch hergestellt, insbesondere auch im Hinblick auf bestimmte Kommunikationsstrecken und zu empfangende Kommunikationsinformationen. Beispielsweise ist es hinsichtlich einer Empfangseinheit einer Magnetresonanzeinrichtung denkbar, über isochrone Kommunikation empfangene Kommunikationsinformationen unmittelbar einer Weiterverarbeitung im selben FPGA zuzuführen, beispielsweise wenigstens einem Auswertungsschritt zu unterziehen. Eine derartige Hochintegration bringt neben reduziertem Herstellungsaufwand auch kürzere Signalwege und somit schnellere Verarbeitungszeiten mit sich. Denkbar sind im Übrigen auch Ausgestaltungen, in denen die komplette Recheneinrichtung integriert mit der Kommunikationsschnittstellenbaugruppe bereitgestellt wird.

Insbesondere kann hierbei vorgesehen sein, dass alle eine paketorientierte und/oder eine isochrone Kommunikation benötigenden Recheneinrichtungen eine diese verbindende Kommunikationseinrichtung der erfindungsgemäßen Art aufweisen. Das bedeutet, die Kommunikation innerhalb der medizinischen Bildgebungseinrichtung kann vereinheitlicht und insbesondere auch standardisiert werden, nachdem die erfindungsgemäß vorgeschlagene Kommunikationsschnittstelle sowohl paketorientierte Kommunikation als auch kanalorientierte, isochrone Kommunikation unterstützt und entsprechende Services bereitstellt. Durch möglichst weitgehenden, konsequenten Einsatz der erfindungsgemäßen Kommunikationseinrichtung wird also die Komplexität der medizinischen Bildgebungseinrichtung nicht nur dadurch reduziert, dass nur noch eine Hardware für die entsprechenden Kommunikationsverbindungen notwendig ist, sondern auch im Hinblick auf die Verwendung einheitlicher Kommunikationsschnittstellen.

Für eine derartige erfindungsgemäße Magnetresonanzeinrichtung kann, wie bereits angedeutet, mit besonderem Vorteil vorgesehen sein, dass von den Kommunikationsschnittstellenbaugruppen zu nutzende Taktgeber einen Grundtakt einer Master-Clock (Master-Taktgeber) nutzen, die im Wesentlichen nicht mit der Magnetresonanzbildgebung interferiert, insbesondere einen Grundtakt von 2,5 MHz. Magnetresonanzeinrichtungen sind äußerst empfindlich gegenüber elektromagnetischen Störungen digitaler Logik. Um die Interferenz mit den analogen Signalen möglichst zu verhindern, wird vorgeschlagen, alle Taktgeber (Clocks) so auszugestalten, dass deren Takt bzw. Frequenz von einer einzigen Master-Clock, also einem Master-Taktgeber, abgeleitet sind. In dieser Ausgestaltung sind mithin keine lokalen Taktgeber oder frei laufende Oszillatoren zulässig. Die verschiedenen Takte bzw. Frequenzen sind dabei zweckmäßigerweise als 2,5 MHz (Grundtakt bzw. Systemtakt) und ganzzahlige Vielfache von 2,5 MHz beschränkt. Steuer- und Datensignale weisen bevorzugt keine sich wiederholenden Muster bei anderen Frequenzen als 2,5 MHz oder ganzzahligen Vielfachen von 2,5 MHz auf. Insbesondere kann in einer Weiterbildung vorgesehen sein, dass eine Scrambler-Einrichtung seitens der Trennungs- und Zusammenführungseinheiten vorgesehen wird, um Datensignale mit potentiellen, sich wiederholenden Mustern, die in nicht erlaubten Frequenzen resultieren, entsprechend zu entstören.

Wie bereits erwähnt, findet in der Magnetresonanzeinrichtung, zumindest innerhalb des Magnetresonanzraums, die Kommunikation bevorzugt über optische Kommunikationsleitungen statt. Beispielsweise können für die Kommunikationsleitungen fiberoptische Vollduplexkabel mit einer einzelnen Glasfaser für jede Kommunikationsrichtung eingesetzt werden, wodurch beispielsweise physikalische Übertragungsraten von 2,5 GBit/s, 5 GBit/s oder auch 10 GBit/s bereitgestellt werden können.

Es sei noch darauf hingewiesen, dass die hier vorgeschlagene Kommunikationsstruktur für die Magnetresonanzeinrichtung zumindest teilweise das bereits diskutierte Daisy Chaining nutzt, so dass mithin zumindest teilweise bevorzugt entsprechende Arbitrierungseinrichtungen, wie beschrieben, eingesetzt werden können.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer Kommunikationseinrichtung einer Ausführungsform einer erfindungsgemäßen medizinischen Bildgebungseinrichtung,
- Fig. 2: die Aufteilung einer verwendeten Datenstruktur, und
- Fig. 3: die Kommunikationsstruktur einer Ausführungsform einer erfindungsgemäßen Magnetresonanzeinrichtung.

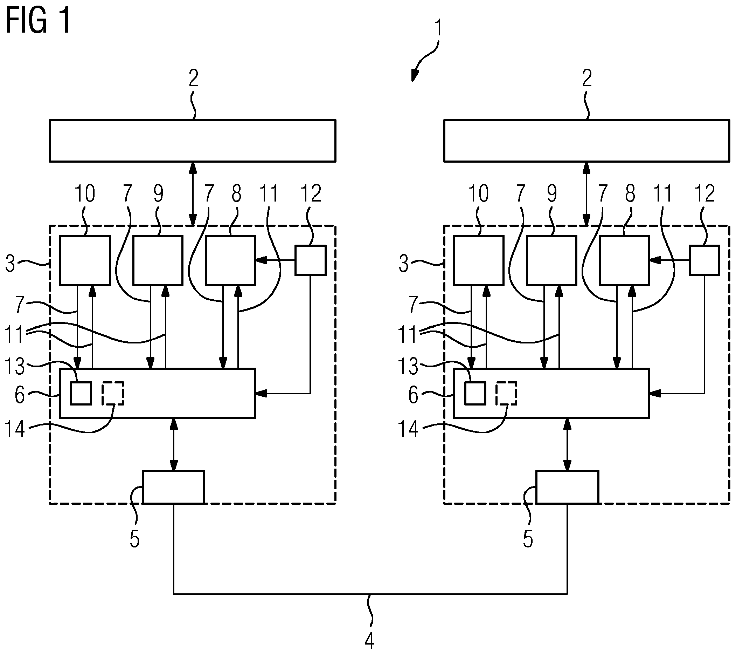

Fig. 1 zeigt eine Prinzipskizze eines Ausführungsbeispiels einer Kommunikationseinrichtung 1 einer erfindungsgemäßen medizinischen Bildgebungseinrichtung, die zur Kommunikation zwischen zwei Recheneinrichtungen 2, die zu Komponenten einer Magnetresonanzeinrichtung als medizinische Bildgebungseinrichtung gehören, dient. Die Kommunikationseinrichtung 1 weist für jede der Recheneinrichtungen 2 eine Kommunikationsschnittstellenbaugruppe 3 auf, wobei die Kommunikationsschnittstellenbaugruppen 3 durch eine Kommunikationsleitung 4 verbunden sind, welche bei elektrischer Datenübertragung als übliches elektrisches Kabel ausgebildet sein kann, innerhalb eines Magnetresonanzraums jedoch bevorzugt als optische Kommunikationsleitung 4 ausgebildet ist, die voll duplexfähig ist, mithin für jede Kommunikationsrichtung eine Glasfaser aufweist.

Im hier dargestellten Fall der optischen Kommunikationsleitung 4 weist die Kommunikationsschnittstellenbaugruppe 3 zunächst einen Optokoppler 5 (optisch-elektronischer Wandler) auf. Kernstück der Kommunikationsschnittstellenbaugruppe 3 ist eine Trennungs- und Zusammenführungseinheit 6, die entsprechend jeweils mit dem Optokoppler 5 verbunden ist.

Die Trennungs- und Zusammenführungseinheit 6 dient zunächst dazu, Kommunikationsinformationen unterschiedlicher Kommunikationsarten, von denen wenigstens eine eine paketorientierte Kommunikation und wenigstens eine eine isochrone Kommunikation ist, zu gemeinsamen, fest aufgeteilten Datenstrukturen vorgegebener, fester Größe zusammenzuführen, die über die einzige Kommunikationsleitung 4 zu übertragen sind, wobei die Datenstrukturen immer mit Abstand genau einer Zykluszeit, beispielsweise 200 ns oder 400 ns, übertragen werden. Die Kommunikationsinformationen werden der Trennungs- und Zusammenführungseinheit 6 über Dateneingänge 7 von entsprechenden Einheiten der Transportschicht bereitgestellt, während die Trennungs- und Zusammenführungseinheit 6 dem Data Link Layer (Sicherungsschicht) zuzuordnen ist. Nachdem vorliegend als Kommunikationsarten über die hier beschriebene gemeinsame Kommunikationsschnittstelle eine isochrone Kommunikation, die einer isochronen Direktkanalverbindung entspricht, eine Streaming-Kommunikation und eine Wahlfreier-Zugriff-Kommunikation (random access communication) bereitgestellt werden sollen, weist die Kommunikationsschnittstellenbaugruppe 3 eine Isochronkommunikationseinheit 8, die über den entsprechenden Dateneingang 7 isochrone Kommunikationsinformation bereitstellt, sowie zwei Paketkommunikationseinheiten 9, 10 jeweils für die Streaming-Kommunikation und die Wahlfreier-Zugriff-Kommunikation bereit. Diese Einheiten sind es letztlich auch, die von den Recheneinrichtungen 2 angesprochen werden; die Trennungs- und Zusammenführungseinheit 6 ist für die Applikationen in den Recheneinrichtungen 2 letztlich "unsichtbar". Mittels der Paketkommunikationseinheiten 9, 10 können verschiedene konkrete paketorientierte Kommunikationsprotokolle realisiert werden, beispielsweise TCP/IP, CAN/CANopen, proprietäre Protokolle und dergleichen.

Die Trennungs- und Zusammenführungseinheit 6 dient im Empfangsfall mithin auch dazu, die entsprechenden Kommunikationsinformationen aus den empfangenen Datenstrukturen wieder abzutrennen und über entsprechende Datenausgänge 11 der Isochronkommunikationseinheit 8 und den Paketkommunikationseinheiten 9, 10 bereitzustellen. Ein Paar von Dateneingang 7 und Datenausgang 11 bildet eine interne Schnittstelle für die entsprechenden, der Kommunikationsort zugeordneten Kommunikationsinformationen.

Nachdem das in Fig. 1 dargestellte Ausführungsbeispiel der Kommunikationseinrichtung 1 in einem Magnetresonanzraum einer Magnetresonanzeinrichtung eingesetzt werden soll, ist auf möglichst weitgehende Störungsfreiheit auch bezüglich der verwendeten elektrischen Signale in den Kommunikationsschnittstellenbaugruppen 3 zu achten. Mithin ist eine Master-Clock 12 als einziger Taktgeber vorgesehen, die mit einer Grundfrequenz von 2,5 MHz betrieben wird. Innerhalb der Kommunikationsschnittstellenbaugruppe 3 sind lediglich ganzzahlige Vielfache dieser Grundfrequenz von 2,5 MHz zulässig, wobei die den verschiedenen Kommunikationsarten zugeordneten Kommunikationseinheiten 8 - 10 durchaus eigene, hier nicht näher dargestellte Taktgeber aufweisen können, wenn jeweils ein eigener Takt bzw. eigene Frequenz verwendet werden soll. Diese Taktgeber nutzen dabei jedoch das Signal der Master-Clock 12.

Die Trennungs- und Zusammenführungseinheit 6 kann auch analysieren, ob durch wiederkehrende Muster in den Kommunikationsinformationen bzw. den entstehenden Datenstrukturen andere Frequenzen entstehen können, wobei sie eine Scrambler-Einrichtung umfassen kann, die im Zweifel für eine Beseitigung solcher störender Frequenzen sorgt bzw. auf der Empfangsseite eine entsprechende Entzerrung vornimmt.

Die Trennungs- und Zusammenführungseinheit 6 weist ferner eine Vorwärtsfehlerkorrektureinrichtung 13 auf, die alle Kommunikationsinformationen hinsichtlich einer Vorwärtsfehlerkorrektur verarbeitet. Im Fall eines Daisy Chaining, bei dem mehrere Recheneinrichtungen 2, die durch jeweilige Kommunikationseinrichtungen 1 verbunden sind, seriell hintereinandergeschaltet sind, kann zudem eine Arbitrierungseinrichtung 14 vorgesehen sein, auf die im Folgenden noch genauer eingegangen werden wird.

Die Kommunikationsschnittstellenbaugruppen 3 können beispielsweise als Steckkarten oder zumindest auf einer gemeinsamen Leiterplatte realisiert sein. Besonders bevorzugt sind zumindest Anteile der Kommunikationsschnittstellenbaugruppen 3 als ein FPGA oder Teil eines FPGAs realisiert, was insbesondere für die Trennungs- und Zusammenführungseinheit 6 sowie die Isochronkommunikationseinheit 8 und die Paketkommunikationseinheiten 9, 10 gilt. Insbesondere ist eine Ausgestaltung denkbar, in der die Einheiten 6, 8 - 10 innerhalb eines einzigen FPGA realisiert sind, der zudem noch zumindest einen Teil der Funktionen der Recheneinrichtung 2 realisiert, mithin einen Teil von deren Rechenleistung bereitstellt. Gerade im Hinblick auf isochrone Kommunikationsinformationen stellt dies einen großen Vorteil dar, da die isochronen Kommunikationsinformationen unmittelbar und ohne zu großen Zeitverzug weiterverarbeitet werden können.

Die Kommunikationseinrichtung 1 ist zur Durchführung des erfindungsgemäßen Verfahrens ausgebildet. Konkret werden also von den Paketinformationseinheiten 9, 10 und der Isochronkommunikationseinheit 8 erhaltene Kommunikationsinformationen der entsprechenden Kommunikationsarten in Datenstrukturen nebst einer Vorwärtsfehlerkorrekturinformation und einem Header zusammengefasst, wobei die Datenstrukturen zyklisch über die Kommunikationsleitung 4 übertragen werden, wobei eine entsprechende Auftrennung der Datenstrukturen inklusive Vorwärtsfehlerkorrektur innerhalb der empfangenden Trennungs- und Zusammenführungseinheit 6 vorgenommen wird. Vorliegend wird eine Master-Slave-Architektur verwendet, in der eine der Trennungs- und Zusammenführungseinheiten 6 als ein Master, die andere als ein Slave wirkt. Paketorientierte Kommunikationsarten werden dabei seitens des Masters initiiert.

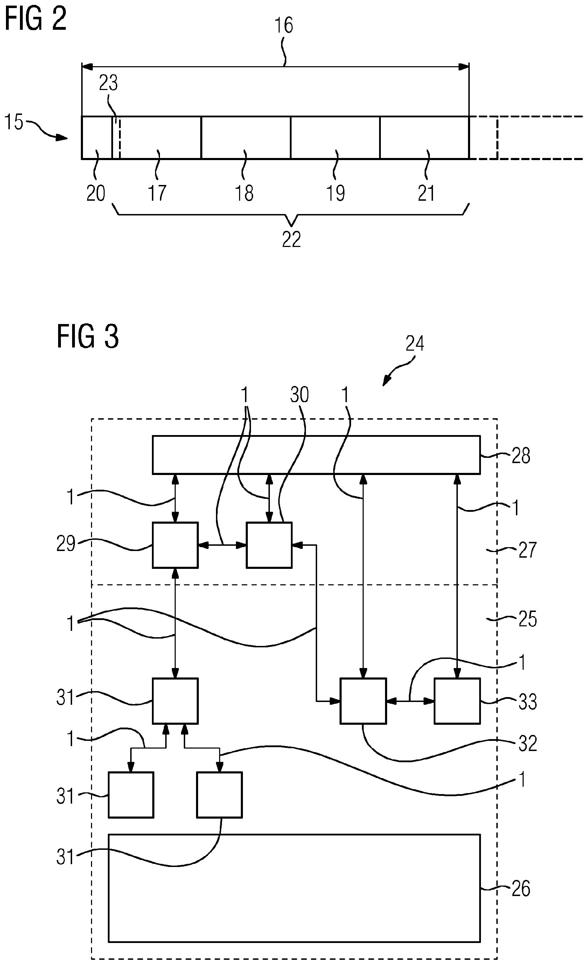

Eine mögliche Aufteilung der Datenstruktur 15 vorgegebener Größe ist in Fig. 2 schematisch gezeigt. Gestrichelt angedeutet ist auch eine nachfolgende Datenstruktur, die dann nach Ablauf der Zykluszeit 16 ausgesendet wird.

Die Datenstruktur 15 umfasst festgelegte Anteile 17, 18 und 19 für Kommunikationsinformationen, wobei der Anteil 17 für Kommunikationsinformationen der Wahlfreier-Zugriff-Kommunikation, der Anteil 18 für Kommunikationsinformationen der Streaming-Kommunikation und der Anteil 19 für Kommunikationsinformationen der isochronen Kommunikation vorgesehen ist. Eingerahmt werden die Anteile 17 - 19 von einem Header 20 und einem Vorwärtsfehlerkorrekturanteil 21 für die Vorwärtsfehlerkorrekturinformation, wie beschrieben. Die Anteile 17, 18, 19 und der Vorwärtsfehlerkorrekturanteil 21 bilden auf der physikalischen Ebene die Nutzdaten 22 (Payload).

In einer konkreten Ausgestaltung kann die Datenstruktur 15 (frame) aus 25 Slots (Nr0 bis Nr24) zu je vier Bytes zusammengesetzt sein. Dem Header kann der erste Slot (Nr0) zugeordnet sein, dem Anteil 17 die nächsten drei Slots, dem Anteil 18 die folgende drei Slot und dem Anteil 19 die nächsten sechzehn Slots. Die zwei abschließenden Slots können für die Vorwärtsfehlerkorrekturinformation im Vorwärtsfehlerkorrekturanteil 21 reserviert sein. Andere Framelängen und/oder andere Aufteilungen der Gesamtbandbreite sind selbstverständlich ebenso denkbar.

Die Kommunikationseinrichtung 1 kann dabei physikalische Datenübertragungsraten von 2,5 GHz/s, 5 GHz/s oder 10 GHz/s (oder darüber hinaus noch höhere Datenraten) realisieren, wobei die entsprechenden Zykluszeiten 400 ns, 200 ns bzw. 100 ns betragen. Denkbar ist auch eine Ausgestaltung, in der die Kommunikationseinrichtung 1 konfigurierbar mehrere Datenübertragungsgeschwindigkeiten bereitstellt, wobei beim Aushandeln der Kommunikationsverbindung eine entsprechende Datenübertragungsrate festgelegt werden kann, die benötigt wird.

Bei der seriellen Datenübertragung der Datenstrukturen 15 ist sichergestellt, dass immer im Abstand einer Zykluszeit 16 ein Header 20 eintrifft, was mithin auch für entsprechende Zeitfenster der Zykluszeit für die entsprechenden anderen Anteile 17, 18, 19 und den Vorwärtsfehlerkorrekturanteil 21 gilt.

Zusammenfassend lässt sich also sagen, dass ein Kommunikationsprotokoll auf physikalischer Ebene bereitgestellt wird, das als ein Punkt-zu-Punkt-serielles Kommunikationsprotokoll bezeichnet werden kann, welches Kommunikationsinformationen dreier verschiedener Kommunikationsarten durch Zeitaufteilungs-Multiplexing aufgrund der Vorwärtsfehlerkorrektur sicher überträgt.

Fig. 2 zeigt ferner auch eine Besonderheit der hier beschriebenen Ausgestaltung. Ein Unteranteil 23, beispielsweise Slot, des Anteils 17 der Wahlfreier-Zugriff-Kommunikation ist zur Realisierung einer Interrupt-Funktion, mittels der der Slave dem Master ein Interrupt senden kann, reserviert.

Es sei an dieser Stelle noch angemerkt, dass für den wahlfreien Zugriff über adressierbare, hier nicht näher dargestellte Register bzw. Speicherbereiche vorgesehen sein kann, dass ein Zugriff erst komplettiert werden muss, bevor ein zweiter Zugriff beginnen kann. Allerdings sind auch Ausgestaltungen, in denen "Split Transactions" möglich sind, denkbar. Ferner ist die hier beschriebene und durch einen Aushandelvorgang erzeugte Kommunikationsverbindung vollständig duplex-bidirektional, so dass beide Seiten gleichzeitig senden und empfangen können.

Fig. 3 zeigt schematisch die Kommunikationsstruktur einer Magnetresonanzeinrichtung 24 als erfindungsgemäße medizinische Bildgebungseinrichtung. In einem Magnetresonanzraum 25 ist dabei, wie grundsätzlich bekannt, eine Hauptmagneteinheit 26 angeordnet, die die eigentlichen bildgebenden Komponenten umfasst. Zudem existiert ein Technikraum 27, in dem eine Steuereinrichtung 28 als Steuerrechner vorgesehen ist. Diese zentrale Steuereinrichtung 28 steuert den Betrieb der Magnetresonanzeinrichtung 24 und kommuniziert dabei direkt oder indirekt mit weiteren Komponenten der Magnetresonanzeinrichtung 24 bzw. deren Recheneinrichtungen 2. Dabei sind als weitere Komponenten im Technikraum 27 hier eine Peripherieeinheit 29 und eine Gradienteneinheit 30 gezeigt. Im Magnetresonanzraum 25 befinden sich neben Peripheriesubeinheiten 31 eine Sendeinheit 32 und eine Empfangseinheit 33. Zwischen dem zentralen Steuerrechner 28 als erste Recheneinrichtung bzw. die erste Recheneinrichtung umfassend und den Recheneinrichtungen der Komponenten Peripherieeinheit 29, Gradienteneinheit 30, Sendeinheit 32 und Empfangseinheit 33 als zweite Recheneinrichtungen ist genauso jeweils eine Kommunikationseinrichtung 1 vorgesehen wie zwischen der Recheneinrichtung der Peripherieeinheit 29 und den Recheneinrichtungen der Peripheriesubeinheiten 31 als dritte Recheneinrichtungen. Weitere Kommunikationseinrichtungen 1 sind zwischen den zweiten Recheneinrichtungen vorgesehen, wobei hier auch ein geschlossener Kommunikationsring vorliegen kann.

Ersichtlich wird bei der in Fig. 3 gezeigten Kommunikationsstruktur wenigstens teilweise Daisy Chaining genutzt, so dass zumindest in der Peripherieeinheit 29, aber auch dann, wenn beispielsweise die Empfangseinheit 33 aus einer digitalen Empfangseinheit und einer analogen Empfangseinheit besteht, die jeweils zwischengeschaltete Recheneinrichtung eine Kommunikationsschnittstellenbaugruppe 3 mit einer Arbitrierungseinrichtung 14 aufweist. Diese stellt die Kommunikationsziele von Kommunikationsinformationen fest und leitet die Kommunikationsinformation bei nicht der eigenen Recheneinrichtung entsprechendem Kommunikationsziel weiter.

Ferner sei angemerkt, dass neben Magnetresonanzdaten an sich eine isochrone Kommunikation auch bei Peripherieeinheiten 29, 31 zweckmäßig sein kann, beispielsweise bei der Aufnahme von physiologischen Messwerten des Patienten oder dergleichen. Die hier beschriebene Kommunikationseinrichtung und mithin Kommunikationsschnittstelle erlaubt mithin eine vereinheitlichte, gleichzeitige paketorientierte und kanalorientierte Kommunikation auf einer einzigen Kommunikationsleitung 4. Insbesondere kann dieselbe Kommunikationsschnittstelle bzw. Kommunikationseinrichtung 1 in der gesamten Magnetresonanzeinrichtung 24 eingesetzt werden, was die Komplexität, Entwicklungsaufwände und dergleichen reduziert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden. Der Schutzumfang der Erfindung wird von den beigelegten Patentansprüchen definiert.

## Patentansprüche

1. Verfahren zur Kommunikation zwischen wenigstens zwei Recheneinrichtungen (2) einer medizinischen Bildgebungseinrichtung, einer Magnetresonanzeinrichtung, über eine einzige Kommunikationsleitung (4),
wobei über jeweils einen eigenen Dateneingang (7) erhaltene, unterschiedlichen Kommunikationsarten zugeordnete Kommunikationsinformationen in über die Kommunikationsleitung (4) zu übertragende Datenstrukturen (15) definierter Größe zusammengeführt werden, wobei die Datenstrukturen (15) zyklisch um eine Zykluszeit (16) beabstandet über die Kommunikationsleitung (4) übertragen werden, und Kommunikationsinformationen der unterschiedlichen Kommunikationsarten aus über die Kommunikationsleitung (4) empfangenen Datenstrukturen (15) extrahiert und an den jeweiligen Kommunikationsarten zugeordnete Datenausgänge (11) ausgegeben werden, wobei die Kommunikationsarten eine erste, paketorientierte Kommunikationsart und eine zweite, isochrone, einer isochronen Direktkanalkommunikation entsprechende Kommunikationsart umfassen,
wobei die Magnetresonanzeinrichtung eine zentrale Steuereinrichtung (28) als erste Recheneinrichtung (2), Recheneinrichtungen (2) wenigstens einer Gradienteneinheit (30), wenigstens einer Sendeinheit (32), wenigstens eine Empfangseinheit (33) und wenigstens einer Peripherieeinheit (29) als zweite Recheneinrichtungen (2) und Recheneinrichtungen (2) wenigstens einer Peripheriesubeinheit (31) als dritte Recheneinrichtungen (2) aufweist, wobei die erste Recheneinrichtung (2), als Master, jeweils über eine der wenigstens einen Kommunikationseinrichtung (1) mit den zweiten Recheneinrichtungen (2), als Slave, verbunden ist und wobei die Peripherieeinheit, als Master, über jeweils eine der wenigstens einen Kommunikationseinrichtung (1) mit den dritten Recheneinrichtungen (2), als Slave, verbunden ist,
**dadurch gekennzeichnet,**
**dass** wenigstens ein vorbestimmter Unteranteil (23) der Datenstruktur (15) zum Übermitteln eines Interrupts von einem Slave an einen Master verwendet wird.

2. Verfahren nach Anspruch 1, **dadurch gekennzeichnet, dass** als erste Kommunikationsart eine Streaming-Kommunikation und/oder eine Wahlfreier-Zugriff-Kommunikation zum Abfragen wenigstens eines Speicherregisters wenigstens einer der Recheneinrichtungen (2) verwendet werden.

3. Verfahren nach Anspruch 2, **dadurch gekennzeichnet, dass** eine parallele Handhabung mehrerer Anfragen in der Wahlfreier-Zugriff-Kommunikation erfolgt.

4. Verfahren nach einem der vorangehenden Ansprüche, **dadurch gekennzeichnet, dass** eine optische Kommunikationsleitung (4) als die Kommunikationsleitung (4) verwendet wird, wobei die Datenstrukturen (15) beidseitig durch Optokoppler (5) umgewandelt werden.

5. Verfahren nach einem der vorangehenden Ansprüche, **dadurch gekennzeichnet, dass** eine Vorwärtsfehlerkorrektur erfolgt, wobei eine auf alle Kommunikationsinformationen aller Kommunikationsarten bezogene Vorwärtsfehlerkorrekturinformation erzeugt und in einen Vorwärtsfehlerkorrekturanteil (21) der Datenstruktur (15) eingefügt wird, wobei die Vorwärtsfehlerkorrekturinformation bei Empfang jeder Datenstruktur (15) ausgewertet wird.

6. Verfahren nach einem der vorangehenden Ansprüche, **dadurch gekennzeichnet, dass** bei Kommunikation zwischen zwei Recheneinrichtungen (2) über eine zwischengelagerte Recheneinrichtung (2) durch diese empfangene Datenstrukturen (15) und/oder Kommunikationsinformationen zu Kommunikationszielen zugeordnet werden und an die Kommunikationsziele bei nicht der eigenen Recheneinrichtung (2) entsprechendem Kommunikationsziel weitergeleitet werden.

7. Medizinische Bildgebungseinrichtung mit mehreren Recheneinrichtungen (2) und wenigstens einer Kommunikationseinrichtung (1) zur Kommunikation zwischen wenigstens zwei Recheneinrichtungen (2) der medizinischen Bildgebungseinrichtung, einer Magnetresonanzeinrichtung (24), aufweisend:
- eine Kommunikationsleitung (4), und
- für jede Recheneinrichtung (2) jeweils eine Kommunikationsschnittstellenbaugruppe (3), die mit der Kommunikationsleitung (4) verbunden ist,
wobei die Kommunikationsschnittstellenbaugruppen (3) jeweils eine Trennungs- und Zusammenführungseinheit (6) zum Zusammenführen von über jeweils einen eigenen Dateneingang (7) erhaltenen, unterschiedlichen Kommunikationsarten zugeordneten Kommunikationsinformationen in über die Kommunikationsleitung (4) zu übertragende Datenstrukturen (15) einer definierten Größe sowie zum zyklischen, um eine Zykluszeit (16) beabstandeten Übertragen der Datenstrukturen (15) über die Kommunikationsleitung (4), und zum Extrahieren von Kommunikationsinformationen der unterschiedlichen Kommunikationsarten aus über die Kommunikationsleitung (4) empfangenen Datenstrukturen (15) der jeweils anderen Kommunikationsschnittstellenbaugruppe (3) sowie zur Weitergabe an den jeweiligen Kommunikationsarten zugeordnete Datenausgänge (11), wobei an ein einer ersten, paketorientierten Kommunikationsart zugeordnetes Dateneingang-Datenausgang-Paar eine Paketkommunikationseinheit (9, 10) zur Bereitstellung und Entgegennahme von Kommunikationsinformationen der ersten Kommunikationsart und an ein einer zweiten, einer isochronen Direktkanalkommunikation entsprechenden Kommunikationsart zugeordnetes Dateneingang-Datenausgang-Paar eine isochrone Daten als Kommunikationsinformation bereitstellende und entgegennehmende Isochronkommunikationseinheit (8) angeschlossen ist,
wobei die medizinische Bildgebungseinrichtung eine zentrale Steuereinrichtung (28) als erste Recheneinrichtung (2), Recheneinrichtungen (2) wenigstens einer Gradienteneinheit (30), wenigstens einer Sendeinheit (32), wenigstens einer Empfangseinheit (33) und wenigstens einer Peripherieeinheit (29) als zweite Recheneinrichtungen (2) und Recheneinrichtungen (2) wenigstens einer Peripheriesubeinheit (31) als dritte Recheneinrichtungen (2) aufweist, wobei die erste Recheneinrichtung (2), als Master, jeweils über eine der wenigstens einen Kommunikationseinrichtung (1) mit den zweiten Recheneinrichtungen (2), als Slave, verbunden ist und wobei die Peripherieeinheit, als Master, über jeweils eine der wenigstens einen Kommunikationseinrichtung (1) mit den dritten Recheneinrichtungen (2), als Slave, verbunden ist,
**dadurch gekennzeichnet,**
**dass** ein Slave ausgelegt ist, wenigstens einen vorbestimmter Unteranteil (23) der Datenstruktur (15) zum Übermitteln eines Interrupts von dem Slave an einen Master zu verwenden.

8. Medizinische Bildgebungseinrichtung nach Anspruch 7, **dadurch gekennzeichnet, dass** die Kommunikationsschnittstellenbaugruppen (3) als ASIC und/oder FPGA und/oder als Teil eines FPGA realisiert sind.

9. Medizinische Bildgebungseinrichtung nach Anspruch 8, **dadurch gekennzeichnet, dass** die Kommunikationsschnittstellenbaugruppen (3) durch einen recheneinrichtungsspezifischen FPGA realisiert sind, der wenigstens einen Teil der Rechenleistung der Recheneinrichtung (2) bereitstellt.

10. Medizinische Bildgebungseinrichtung nach Anspruch 8 oder 9, **dadurch gekennzeichnet, dass** sie zur Durchführung eines Verfahrens nach einem der Ansprüche 1 bis 6 ausgebildet ist.

11. Medizinische Bildgebungseinrichtung nach Anspruch 7, **dadurch gekennzeichnet, dass** von den Kommunikationsschnittstellenbaugruppen (3) zu nutzende Taktgeber eine Grundfrequenz einer Master-Clock (12) von 2,5 MHz nutzen.

12. Medizinische Bildgebungseinrichtung nach Anspruch 7, **dadurch gekennzeichnet, dass** die zweiten Recheneinrichtungen (2) wenigstens teilweise untereinander durch wenigstens eine der wenigstens einen Kommunikationseinrichtung (1) verbunden sind.

## Claims

1. Method for communication between at least two computing facilities (2) of a medical imaging facility, a magnetic resonance facility, via a single communication line (4), wherein communication information assigned to different communication types received in each case via a dedicated data input (7) is merged to form data structures (15) of a defined size to be transmitted via the communication line (4), wherein the data structures (15) are transmitted cyclically with an interval of one cycle time (16) via the communication line (4) and communication information of the different communication types is extracted from data structures (15) received via the communication line (4) and output at data outputs (11) assigned to the respective communication types, wherein the communication types comprise a first packet-oriented communication type and a second isochronous communication type corresponding to isochronous direct channel communication, wherein the magnetic resonance facility has a central control facility (28) as a first computing facility (2), computing facilities (2) of at least one gradient unit (30), at least one transmit unit (32), at least one receive unit (33) and at least one peripheral unit (29) as second computing facilities (2) and computing facilities (2) of at least one peripheral subunit (31) as third computing facilities (2), wherein the first computing facility (2), as a master, is connected in each case via one of the at least one communication facility (1) to the second computing facilities (2), as a slave, and wherein the peripheral unit, as a master, is connected via in each case one of the at least one communication facility (1) to the third computing facilities (2), as a slave,
**characterised in that**
at least one prespecified subportion (23) of the data structure (15) is used to transfer an interrupt from a slave to a master.

2. Method according to claim 1, **characterised in that** streaming communication and/or random access communication for requesting at least one storage register of at least one of the computing facilities (2) are used as a first communication type.

3. Method according to claim 2, **characterised in that** parallel handling of a plurality of requests takes place in the random access communication.

4. Method according to one of the preceding claims, **characterised in that** an optical communication line (4) is used as the communication line (4), wherein the data structures (15) are converted on both sides by optocouplers (5) .

5. Method according to one of the preceding claims, **characterised in that** forward error correction takes place, wherein forward-error-correction information, based on all communication information of all communication types, is generated and inserted in a forward-error-correction portion (21) of the data structure (15), wherein the forward-error-correction information is evaluated on the reception of each data structure (15).

6. Method according to one of the preceding claims, **characterised in that**, in the case of communication between two computing facilities (2) via an intermediate computing facility (2), data structures (15) and/or communication information received thereby are assigned to communication targets and forwarded to the communication targets if a communication target does not correspond to the dedicated computing facility (2).

7. Medical imaging facility with a plurality of computing facilities (2) and at least one communication facility (1) for communication between at least two computing facilities (2) of the medical imaging facility, a magnetic resonance facility (24), having:
- a communication line (4) and
- for each computing facility (2) in each case a communication-interface assembly (3) connected to the communication line (4),
wherein the communication interface assemblies (3) in each case a separating-and-merging unit (6) for merging communication information assigned to different communication types received in each case via a dedicated data input (7) into data structures (15) of a defined size to be transmitted via the communication line (4) and for the cyclic transmission of the data structures (15) with an interval of one cycle time (16) via the communication line (4) and for the extraction of communication information of the different communication types from data structures (15) of the respective other communication-interface assembly (3) received via the communication line (4) and forwarding to data outputs (11) assigned to the respective communication types, wherein a packet communication unit (9, 10) for the provision and reception of communication information of the first communication type is connected to a data-input-data-output pair assigned to a first packet-oriented communication type and an isochronous communication unit (8) providing and receiving isochronous data as communication information is connected to a data-input-data-output pair assigned to a second communication type corresponding to isochronous direct channel communication,
wherein the medical imaging facility has a central control facility (28) as a first computing facility (2), computing facilities (2) of at least one gradient unit (30), at least one transmit unit (32), at least one receive unit (33) and at least one peripheral unit (29) as second computing facilities (2) and computing facilities (2) of at least one peripheral subunit (31) as third computing facilities (2), wherein the first computing facility (2), as a master, is connected in each case via one of the at least one communication facility (1) to the second computing facilities (2), as a slave, and wherein the peripheral unit, as a master, is connected via in each case one of the at least one communication facility (1) to the third computing facilities (2), as a slave,
**characterised in that**
a slave is designed to use at least one prespecified subportion (23) of the data structure (15) to transfer an interrupt from the slave to a master.

8. Medical imaging facility according to claim 7, **characterised in that** the communication interface assemblies (3) are implemented as ASIC and/or FPGA and/or as part of an FPGA.

9. Medical imaging facility according to claim 8, **characterised in that** the communication interface assemblies (3) are implemented by a computing-facility-specific FPGA that provides at least one part of the computing power of the computing facility (2).

10. Medical imaging facility according to claim 8 or 9, **characterised in that** it is embodied to carry out a method according to one of claims 1 to 6.

11. Medical imaging facility according to claim 7, **characterised in that** clock-pulse generators to be used by the communication interface assemblies (3) use a fundamental frequency of a master clock (12) of 2.5 MHz.

12. Medical imaging facility according to claim 7, **characterised in that** the second computing facilities (2) are at least partially connected to one another by at least one of the at least one communication facility (1).

## Revendications

1. Procédé de communication entre au moins deux dispositifs (2) informatiques d'un dispositif d'imagerie médicale, un dispositif par résonnance magnétique, par une seule ligne (4) de communication,
dans lequel on rassemble, en des structures (15) de données de grandeur définie à transmettre par la ligne (4) de communication, des informations de communication obtenues par respectivement une entrée (7) de données propre et associées à des types de communication différents, dans lequel on transmet les structures (15) de données cycliquement à distance d'un temps (16) de cycle par la ligne (4) de communication et on extrait des informations de communication des types de communication différents de structures (15) de données reçues par la ligne (4) de communication et on les envoie à des sorties (11) de données associées aux types de communication respectifs, dans lequel les types de communication comprennent un premier type de communication à orientation paquet et un deuxième type de communication isochrone, correspondant à une communication isochrone par canal direct, dans lequel le dispositif par résonnance magnétique a une unité (28) centrale de commande comme premier dispositif (2) informatique, des dispositifs (2) informatiques d'au moins une unité (30) de gradient, au moins une unité (32) d'émission, au moins une unité (33) de réception et au moins une unité (29) périphérique comme deuxièmes dispositifs (2) informatiques et des dispositifs (2) informatiques d'au moins une sous-unité (31) périphérique comme troisièmes dispositifs (2) informatiques, dans lequel le premier dispositif (2) informatique est relié comme maître respectivement par l'un des au moins un dispositif (1) de communication aux deuxièmes dispositifs (2) informatiques, comme esclaves, et dans lequel l'unité périphérique est reliée comme maître par respectivement par au moins l'un des au moins un dispositif (1) de communication aux troisièmes dispositifs (2) informatiques comme esclaves,
**caractérisé**
**en ce que** l'on utilise au moins une sous-proportion (23) définie à l'avance de la structure (15) de données pour la transmission d'une interruption d'un esclave à un maître.

2. Procédé suivant la revendication 1, **caractérisé en ce que** l'on utilise, comme premier type de communication, une communication en streaming et/ou une communication à accès à liberté de choix pour demander au moins un registre de mémoire d'au moins l'un des dispositifs (2) informatiques.

3. Procédé suivant la revendication 2, **caractérisé en ce qu'**un traitement parallèle de plusieurs demandes a lieu dans la communication à accès à liberté de choix.

4. Procédé suivant l'une des revendications précédentes, **caractérisé en ce que** l'on utilise une ligne (4) optique de communication comme ligne (4) de communication, les structures (15) de données étant transformées des deux côtés par optocoupleur (5) .

5. Procédé suivant l'une des revendications précédentes, **caractérisé en ce que** l'on effectue une correction d'erreur sans voie de retour, dans lequel on produit une information de correction d'erreur sans voie de retour rapportée à toutes les informations de communication de tous les types de communication et on l'insère dans une proportion (21) de correction d'erreur sans voie de retour de la structure (15) de données, l'information de correction d'erreur sans voie de retour étant exploitée à la réception de chaque structure (15) de données.

6. Procédé suivant l'une des revendications précédentes, **caractérisé en ce que**, lors de la communication entre deux dispositifs (2) informatiques par un dispositif (2) informatique interposé, on associe des structures (15) de données et/ou des informations de communication reçues par celui-ci à des buts de communication et on les achemine aux buts de communication pour le but de communication ne correspondant pas à son dispositif (2) informatique propre.

7. Dispositif d'imagerie médicale ayant plusieurs dispositifs (2) informatiques et au moins un dispositif (1) de communication pour la communication entre au moins deux dispositifs (2) informatiques du dispositif d'imagerie médicale, un dispositif (24) par résonnance magnétique, comportant :
- une ligne (4) de communication, et
- pour chaque dispositif (2) informatique respectivement un module (3) d'interface de communication, qui est relié à la ligne (4) de communication,
dans lequel les modules (3) d'interface de communication ont respectivement une unité (6) de séparation et de regroupement, pour le regroupement d'informations de communication, obtenues respectivement par une entrée (7) de données propre et associées à des types de communication différents, en des structures (15) de données d'une dimension définie à transmettre par la ligne (4) de communication, ainsi que pour la transmission cyclique à distance d'un temps (16) de cycle des structures (15) de données par la ligne (4) de communication et pour l'extraction d'informations de communication des types de communication différents des structures (15) de données reçues par la ligne (4) de communication des respectivement autres modules (3) d'interface de communication, ainsi que pour l'acheminement aux sorties (11) de données associées aux types de communication respectifs, dans lequel est raccordé à une paire entrée de données - sortie de données associée à un premier type de communication orienté par paquets, une unité (9, 10) de communication par paquets pour la mise à disposition et la réception d'informations de communication du premier type de communication et à une deuxième paire d'entrée de données - sortie de données associée à un type de communication isochrone correspondant à une communication par canal direct, une unité (8) de communication isochrone réceptrice et mettant à disposition une donnée isochrone comme information de communication,
dans lequel le dispositif d'imagerie médicale a une unité (28) centrale de commande comme premier dispositif (2) informatique, des dispositifs (2) informatiques d'au moins une unité (30) de gradient, au moins une unité (32) d'émission, au moins une unité (33) de réception et au moins une unité (29) périphérique comme deuxièmes dispositifs (2) informatiques et des dispositifs (2) informatiques d'au moins une sous-unité (31) périphérique comme troisièmes dispositifs (2) informatiques, dans lequel le premier dispositif (2) informatique est relié comme maître respectivement par l'un des au moins un dispositif (1) de communication aux deuxièmes dispositifs (2) informatiques et dans lequel l'unité périphérique est reliée comme maître par respectivement par au moins l'un des au moins un dispositif (1) de communication aux troisièmes dispositifs (2) informatiques comme esclaves, **caractérisé**
**en ce qu'**un esclave est conçu pour utiliser une sous-proportion (23) définie à l'avance de la structure (15) de données pour la transmission de l'interruption de l'esclave à un maître.

8. Dispositif d'imagerie médicale suivant la revendication 7, **caractérisé en ce que** les modules (3) d'interfaces de communication sont réalisés sous la forme classique ou de FPGA et/ou comme parties d'un FPGA.

9. Dispositif d'imagerie médicale suivant la revendication 8, **caractérisé en ce que** les modules (3) d'interfaces de communication sont réalisés par un FPGA spécifique au dispositif informatique, qui met à disposition au moins une partie de la puissance de calcul du dispositif (2) informatique.

10. Dispositif d'imagerie médicale suivant la revendication 8 ou la revendication 9, **caractérisé en ce qu'**il est constitué pour effectuer un procédé suivant l'une des revendications 1 à 6.

11. Dispositif d'imagerie médicale suivant la revendication 7, **caractérisé en ce que** des horloges à utiliser par les modules (3) d'interfaces de communication utilisent une fréquence fondamentale d'une horloge (12) maître de 2,5 MHz.

12. Dispositif d'imagerie médicale suivant la revendication 7, **caractérisé en ce que** les deuxièmes dispositifs (2) informatiques sont reliés au moins en partie entre eux par au moins l'un des au moins un dispositif (1) de communication.
